# EUROPEAN PATENT SPECIFICATION

(11) **EP 1 778 863 B1**
(45) Date of publication and mention of the grant of the patent: **08.06.2016**
(21) Application number: 05761305.1
(22) Date of filing: 28.06.2005
(51) Int. Cl.: C12Q 1/68

(54) **INTEGRATED NUCLEIC ACID ANALYSIS**
INTEGRIERTE NUKLEINSÄUREANALYSE
ANALYSE D'ACIDE NUCLÉIQUE INTÉGRÉE

(30) Priority: 29.06.2004 FI 20045248
(43) Date of publication of application: 02.05.2007
(73) Proprietor: WALLAC OY, 20750 Turku (FI)
(72) Inventor: OLLIKKA, Pia, 20660 Littoinen (FI); YLIKOSKI, Alice, 21500 Piikkiö (FI)
(74) Representative: Kolster Oy Ab
(86) International application number: PCT/FI2005/050245
(87) International publication number: WO 2006/000648

(56) References cited:
- WO-A-00/12675
- WO-A2-01/92569
- US-A- 5 382 511
- US-B1- 6 168 922
- SJÖROOS M ET AL: "Solid-phase PCR with hybridization and time-resolved fluorometry for detection of HLA-B27." CLINICAL CHEMISTRY MAR 2001, vol. 47, no. 3, March 2001 (2001-03), pages 498-504, XP002556362 ISSN: 0009-9147
- STRIZHKOV BORIS N ET AL: "PCR amplification on a microarray of gel-immobilized oligonucleotides: Detection of bacterial toxin- and drug-resistant genes and their mutations" BIOTECHNIQUES, INFORMA LIFE SCIENCES PUBLISHING, WESTBOROUGH, MA, US, vol. 29, no. 4, 1 October 2000 (2000-10-01), pages 844-857, XP002167651 ISSN: 0736-6205
- NURMI J ET AL: "High-throughput genetic analysis using time-resolved fluorometry and closed-tube detection." ANALYTICAL BIOCHEMISTRY 15 DEC 2001, vol. 299, no. 2, 15 December 2001 (2001-12-15), pages 211-217, XP002556363 ISSN: 0003-2697
- CAGGANA M ET AL: "Rapid, efficient method for multiplex amplification from filter paper." HUMAN MUTATION 1998, vol. 11, no. 5, 1998, pages 404-409, XP002556364 ISSN: 1059-7794
- ROY REENA ET AL: "Infrared fluorescent detection of D1S80 alleles from blood and body fluid collected on IsoCodeTM devices" BIOTECHNIQUES, INFORMA LIFE SCIENCES PUBLISHING, WESTBOROUGH, MA, US, vol. 23, no. 5, 1 November 1997 (1997-11-01), pages 942-945, XP008114926 ISSN: 0736-6205
- NURMI J. ET AL: 'High-Throughput Genetic Analysis Using Time-Resolved Fluorometry and Closed-Tube Detection' ANALYTICAL BIOCHEMISTRY vol. 299, 10 November 2001, pages 211 - 217, XP002556363
- SJÖROOS M. ET AL: 'Solid-Phase PCR with Hybridization and Time-resolved Fluorometry for Detection of HLA-B27' CLINICAL CHEMISTRY vol. 47, no. 3, March 2001, pages 498 - 504, XP002556362
- KLINE M. ET AL: 'Polymerase Chain Reaction Amplification of DNA from Aged Blood Stains: Quantitative Evaluation of the "Suitability for Purpose" of Four Filter Papers as Archival Media' ANALYTICAL CHEMISTRY vol. 74, no. 8, 15 April 2002, pages 1863 - 1869, XP008116287
- VALIMAA L ET AL: "Detection of HLA-B27 alleles by group-specific amplification and time-resolved fluorometry", JOURNAL OF IMMUNOLOGICAL METHODS, ELSEVIER SCIENCE PUBLISHERS B.V.,AMSTERDAM, NL, vol. 219, no. 1-2, 1 October 1998 (1998-10-01), pages 131-137, XP004142547, ISSN: 0022-1759, DOI: 10.1016/S0022-1759(98)00137-9
- ARVE ULVIK ET AL: "Single nucleotide polymorphism (SNP) genotyping in unprocessed whole blood and serum by real-time PCR: Application to SNPs affecting homocysteine and folate metabolism", CLINICAL CHEMISTRY, AMERICAN ASSOCIATION FOR CLINICAL CHEMISTRY, WASHINGTON, DC, vol. 47, no. 11, 1 November 2001 (2001-11-01), pages 2050-2053, XP002461353, ISSN: 0009-9147

## Description

### Field of the Invention

The present invention relates to an integrated method of nucleic acid analysis, and more particularly to a simplified sample pre-treatment, which renders the method more easily automated. The method of the present invention provides minimized risks related to contamination due to minimal handling of the samples.

### Background of the Invention

As a result of the rapidly developing genomic testing, whole nucleic acid analysis is an increasing task in many genetic laboratories. The polymerase chain reaction (PCR) is a well established method for amplifying nucleic acid sequences, and the method is routinely used in numerous application areas, such as microbiological testing, expression studies, determination of genetic variation in population, and genetic testing, forensics and food and environmental testing. Testing of nucleic acids using PCR generally involves three steps: sample preparation, amplification and detection. However, the processes for performing the tests used today are often laborious. The current trend is towards simplified assays allowing automation for nucleic acid analysis.

The increased number of tests to be run raises a need for costeffective operations based on integration and automation of the assay procedures, and the automation of the whole DNA analysis is an increasing task in many genetic laboratories. Although several attempts to perform DNA-analysis as high throughput assays has been described, the logistics of sample handling from sample preparation and pre-treatment to the ultimate analysis still requires manual handling and physical transportation of the samples. In addition, the need to physically transfer amplified DNA samples within the lab poses a serious contamination risk.

Sjöroos et al. (Clin. Chem. 2001, 47:498-504) and Välimaa et al. (J. Immunol. Meth, 1998, 219:131-137) disclose methods of performing multiplex PCR and hybridization detection in a single well of a microtiter plate Although these approaches reduce the hands-on time as compared to conventional, non-integrated methods, they still involve manual handling and require adding and removing various reagents.

International Patent Publication WO 00/12675 discloses a device, which integrates nucleic acid extraction, specific target_amplification and detection into a single device. However, the device consists of a plurality of separate reaction chambers for performing different steps of nucleic acid extraction, amplification and detection. The device is, thus, not fully integrated.

Strizhkov et al. (Biotechniques 2000, 29:844-857) discloses use of microchip gel pads for nucleic acid amplification and detection. This method does not involve integrated nucleic acid extraction but requires isolated DNA be used as a starting material. Furthermore, the system requires manual handling as the hybridization chamber is disassembled after amplification, and the microchip has to be washed and air-dried manually.

One type of a simplified assay that reduces sample handling is provided by so-called closed-tube assays. In closed-tube assays, the PCR product is analyzed in the amplification tube by a homogeneous method, such as TaqMan^{®} (US Patent No. 5210015) or by molecular beacons (US Patent No. 5118801). These approaches allow integration of amplification and detection. However, they lack integration of sample preparation with amplification and detection.

Nurmi et al. (Analyt. Biochem 2001, 299:211-217) discloses a method for high-throughput genetic analysis using time-resolved fluorometry and closed tube. detection. However, the method lacks integrated nucleic acid extraction.

Pre-treatment of the sample is required to remove common inhibitors to nucleic acid amplification that may be present in samples from biological sources. Inhibitors to amplification include, e.g. naturally occurring chelating agents, enzymes and/or proteins that can damage either nucleic acid templates or PCR polymerases used in the amplification reactions. In addition, the common anticoagulants that are used to treat whole blood samples can interfere with nucleic acid amplification reactions.

Numerous technologies have been developed to purify nucleic acids from biological samples but all available procedures are time-consuming and labour intensive. There are several automated stations for sample preparation available on the market, based on silica-chaotrop extraction (US Patent No. 5 234 809) columns, such as QIAamp^{®}, or the like, and various magnetic bead systems. A different approach is the FTA^{®} Technology (US Patent No. 5 496 562) that lyses cell membranes as soon as the sample is applied onto a coated filtration matrix allowing immobilization of nucleic acids onto the matrix. After washing, the nucleic acids can be released in a manner that enables them to be amplified by PCR.

Ulvik et al. (Clin. Chem. 2001, 47:2050-2053) discloses a method of SNP genotyping in unprocessed whole blood and serum by real-time PCR, wherein the whole blood samples are deposited at the bottom of the 96-well plates and dried. Real-time PCR using a TaqMan® assay is then run in a closed-tube assay.

Another approach is represented by the development of different solid matrices for collecting, transporting, storing and purifying biological samples, such as clinical whole blood, saliva or faecal samples, for nucleic acid analysis. US Patent No. 5 807 527 describes a solid medium for long term storage of blood DNA, which comprises a composition, which protects against degradation of DNA, a protein denaturing agent and a free radical trap.

Another example of this approach is the described in US Patent No. 5939259, which discloses an absorbent material, which does not bind nucleic acids irreversibly which is impregnated with a chaotropic salt.

In these known technologies, the DNA of stored blood samples is extracted from the medium before performing PCR, or the DNA is used in PCR in situ on the solid medium after extensive purification, as described in US Patent No. 5 807 527. Extraction or elution of DNA from the medium requires a multi-step procedure with special solutions and incubations.

Known technologies using solid media for collecting, storing and purifying DNA thus involve multi-step procedures, some using several separate vials and solutions, for generating a sample useful for amplification and detection. Moreover, certain of these methods unnecessarily produce waste.

### Brief Description of the Drawings

Figure 1 is a graphical presentation of the result of the comparative test described in Example 1, analyzing the effect of pre-washing dried samples on collection paper.
Figure 2 is a graphical presentation of the result of the test described in Example 2, where the release of DNA from dried samples, amplification and detection of amplified DNA is performed in a single reaction vial.
Figure 3 is a graphical presentation of the result of the test described in Example 3, where the release of DNA from dried samples, amplification and detection of amplified DNA is performed in a single reaction vial, and where all necessary reagents were added simultaneously.
Figure 4 is a graphical presentation of the result of the test described in Example 4, where a whole blood sample is applied to an absorbent matrix in the reaction vial, and amplification and detection of amplified DNA is performed in the same reaction vial.
Figure 5 is a graphical presentation of the result of the test described in Example 5, where all necessary reagents were provided in dried form in the reaction vial.

### Brief Description of the Invention

The present invention relates to a method for analyzing a target nucleic acid in a biological sample, comprising the following steps of i) transferring an absorbent matrix to a single-compartment reaction vial; ii) providing a biological sample containing said target nucleic acids on or applying it onto said absorbent matrix; iii) providing in said reaction vial a reagent mixture for amplifying said nucleic acids; iv) providing in said reaction vial a detection reagent mixture; v) sealing said reaction vial; vi) performing an amplification reaction; and vii) detecting the amplified nucleic acids, wherein steps vi) and vii) are performed in said vial without removing said matrix from the reaction vial and without opening said reaction vial.

More specifically the present invention relates to a method for analyzing a target nucleic acid in a biological sample, wherein the amplification and detection steps are performed in sealed reaction vials without adding or removing any reagents or other components after the amplification step.

The present invention further relates to a method wherein said sample is applied onto the matrix immediately prior to transferring the matrix to said reaction vial, without drying and/or storage.

The present invention further relates to a method wherein said matrix is transferred in the reaction vial prior to applying the sample directly onto the matrix.

The present invention further relates to a method wherein at least part of said amplification reagents and detection mixtures are provided in dried form prior to the addition of the sample, and said method including an optional further step of adding water prior to performing the amplification step.

### Detailed Description of the Invention

The present invention provides an integrated method for analyzing nucleic acids contained in a biological sample, comprising a simplified sample pre-treatment, amplification of the target nucleic acid to be analyzed, and detection of said target sequence. Due to the simplified sample pre-treatment, the method allows automation and integration of sample preparation, amplification and analysis of nucleic acids.

The present invention is based on the surprising finding that the amplification and detection steps may be performed on samples collected and, optionally, stored on solid media without prior extraction, washing or elution of the nucleic acids. Only a short incubation to inactive inhibitors and to release the nucleic acids is needed.

Inactivation of inhibitors and denaturation of disturbing proteins is achieved by use of solid matrices based on any porous or absorbing material, e.g. cellulose based filter paper or wadding, cotton wad or fabric, or synthetic plastic material. Suitable matrices for use in the method according to the present invention include absorbent materials which bind proteinaceous material, but do not bind nucleic acids irreversibly, and which are impregnated with e.g., a base, an acid, reducing agents, chaotropic salts, detergents, or other agents to denature natural inhibitors within the sample. Solid matrix material suitable for use in the present invention may be provided as flat sheets, swabs, tablets, pellets or beads, or as a mesh or lattice.

Such solid matrix materials are readily available, and include e.g. the paper matrix provided by Schleicher&Schuell under the trade marks 903™ Specimen Collection paper or IsoCode^{®} for collecting, storing, transporting and purifying DNA.

In a preferred embodiment of the present invention the biological sample is provided on such an absorbent matrix. At least a portion of the matrix containing the sample is transferred to the reaction vial, together with reagents for amplification and detection. The reaction vial is then sealed and moved to a device for performing the amplification reaction. The DNA of the sample is released by a brief heat-treatment. Optionally, if the reagents are not heat stable, e.g. in case of isothermal amplification, the DNA release may be performed at ambient temperature. Surprisingly, after amplification the detection may be performed without opening the reaction vial or removing said matrix.

It has also surprisingly been found, that the sample may be applied onto the matrix immediately prior to use. Thus, in a preferred embodiment of the present invention, the sample is applied onto a suitable solid matrix, whereafter at least a portion of the matrix is transferred to the reaction vial, without prior drying of the matrix. After addition of necessary reagents, the amplification and detection steps are performed.

It has further, surprisingly, been found, that the solid matrix may be provided in the reaction vial and that the sample may be applied directly onto the matrix, and necessary reagents added, once the sample has been applied. Thereafter the amplification and detection steps are performed in the sealed vial.

The term "amplification" is meant to include any method for amplifying nucleic acids known in the art, either thermal cycling methods such as polymerase chain reaction (PCR; US Patent 4 683 202), reverse transcriptase PCR, (US Patent No. 5310652), and ligase chain reaction (LCR; U.S. Patent No. 5 185 243), and any variations thereof, or isothermal methods, such as Q-Beta replicase technology (U.S. Patent No. 4 786 600), nucleic acid sequence based amplification (NASBA; U.S. Patent No. 5409818), transcription mediated amplification (TMA; U.S. Patent Nos. 5399491), strand displacement amplification (SDA; US Patent No. 5455166) and multiple displacement amplification (MDA; US Patent No. 6124120). One preferred method of amplification is asymmetric PCR, described by Innis et al., PNAS 85(24), 1988: 9436-40, which generates single stranded amplification products.

Other known amplification methods may also be useful in the integrated analysis method according to the present invention. The amplification reagents and the amplification procedure may thus vary according to the method of choice.

The detection step is performed by any suitable detection method of choice. Thus the detection reagents may vary according to the method of choice. Suitable detection methods are based on e.g. intercalating reagents to detect the accumulation of amplification product or to perform melting temperature analysis, or labelled primers, or specific primer extension in which directly or indirectly labelled nucleotides are incorporated to the amplification product, or methods in which the directly or indirectly labelled detection probes are degraded during extension, or hybridization with directly or indirectly labelled probes. The detection may be based on e.g. fluorescence resonance energy transfer, fluorescence quenching or environmentally sensitive labels. Suitable detection methods may also include imaging of array of spots or beads by confocal scanning or evanescent wave. The detection step is preferably performed in a closed-tube format without removing the matrix or unreacted components, in order to avoid post-amplification handling.

One preferred detection method is based on hybridization using labelled probes that recognize the amplified target nucleic acids. In such a method, the hybridization, and thus labelling of the target nucleic acids, is achieved simply by lowering the temperature after amplification to hybridization temperature, if necessary, whereafter the un-reacted labelled probe is quenched by a complementary probe. Detection of formed labelled hybrids may be performed on the reaction vial, without removing the matrix or other unreacted components. Thus the reaction vials remain unopened after performing the amplification reaction.

In a preferred embodiment of the present invention, the method is further simplified in a way that at least part of the reagents necessary for performing the amplification and detection steps, is provided in the reagent vial in dried form prior to the addition of the sample to be analyzed. Optionally, the absorbent matrix may also be provided in the reaction vial. Such a simplified, integrated assay format minimizes the manual handling, is easily automated and decreases the risk of contamination, as the reaction vials may be sealed immediately after providing the sample and, if necessary, adding water.

A variety of samples can be analyzed using the methods of the invention for preparing a sample for molecular analysis. Such samples include bodily fluids, such as whole blood, saliva, sputum, urine, faecal, peritoneal and pleural fluids; lavations, such as bronchoalveolar, nasal, cervical and intestinal samples; aspiration or biopsy samples; cell cultures or microbial cultures. Specific non-limiting examples include whole blood and saliva samples. The biological samples may also be samples taken from food or environmental samples. In addition to complex biological samples, the method of the present invention is equally suitable for amplifying and analysing pre-treated, purified samples, for example samples where DNA and/ or RNA has already been extracted and thereafter stored on matrix.

In a preferred embodiment, the method of the present invention is carried out as follows. The biological sample, such as whole blood, is provided dried on an absorbent matrix sheet or stick, or, optionally, applied to such a matrix by the user, and is allowed to dry onto the matrix. After drying, at least a portion is transferred to the reaction vial. The amplification reagents, comprising primers, nucleotides, polymerase and necessary buffers are added, as well as the detection mixture, comprising directly or indirectly labelled nucleotides or probes, and optionally secondary labels, or intercalators. The vial is then sealed and moved to a thermal cycler. The DNA of the samples is released by a brief heat-treatment. The amplification proceeds after the pre-heating. After amplification, the presence and/or composition of the nucleic acid is detected through the seal on the vial without removing the matrix or even opening the sealed vials at any stage.

In another, highly preferred embodiment, the method of the present invention is carried out as a multi-vial assay. The method is performed using a kit containing dried reagents for amplification and analysis, as well as an absorbent matrix, either as a disk at the bottom of the vials, or as a mesh at least partly filling the vials. The sample to be analyzed is applied, without pre-treatment, directly onto the matrix in the reaction vial. Water is then dispensed into the vials and the vials are sealed and moved to a thermal cycler. The DNA of the samples is released by a brief heat-treatment. The amplification proceeds after the pre-heating. After amplification, the presence and/or composition of the nucleic acid is then detected through the seal of the vials, without removing the matrix. A suitable, readily available format for a kit according to the present invention is e.g. a multi-well plate.

The following examples are given to further illustrate preferred embodiments of the present invention, but are not intended to limit the scope of the invention. It will be obvious to a person skilled in the art that, as the technology advances, the inventive concept can be implemented in various ways. The invention and its embodiments are not limited to the examples described above but may vary within the scope of the claims.

### Examples

### Example 1

### Release of nucleic acids from dried sample disks

This example shows how the elution of dried blood and saliva disks recommended by manufacturer can be simplified. The recommended pre-wash is omitted and it is shown that the release of nucleic acids can be performed effectively also at room temperature. The release is automated by use of filter plates and automated filtration.

First, whole blood and saliva was collected from a volunteer. The samples were applied onto IsoCode^{®} (Schleicher & Schuell) collection paper and allowed to dry. For analysis, eight 1.5-mm disks were punched and the nucleic acids were eluted according to manufacturer's instructions, i.e. prewash with 500 µl of distilled water followed by incubation at + 95°C for 30 min in 135 µl of distilled water. Another eight disks were incubated without prewash at + 95°C for 30 min. After incubation, the supernatant was analysed. The automated release was demonstrated by punching a 1.5-mm disk together with 40 µl of distilled water into a vial in a 96-well filtration plate, and the samples were incubated at room temperature for 30 min. After incubation, the filter plate was aligned with reaction plate and the supernatant was transferred into the reaction plate by pressure from top of the filter plate.

The closed-tube PCR amplification and detection reaction was performed in a total volume of 50 µl and the final concentration in the reaction mixture was following: 1 x DyNAzyme™ buffer (Finnzymes), 0.2 mM dNTP's, 2.5 mM MgCl2, 0.1 µM CFTR exon 10 forward primer (5' AAG CAC AGT GGA AGA ATT TC 3'), 0.1 µM CFTR exon 10 reverse primer (5' CTC TTC TAG TTG GCA TGC T 3'), 0.02 U/µl DyNAzyme™ enzyme (Finnzymes), 20 nM analyte specific oligonucleotide probe (5' TAA AGA AAA TAT CAT CTT TGG TGT TTC CTA TAA 3') labelled at its 5' end with a stable fluorescent W14054 Tb chelate (Wallac) and 0.4 µM quencher probe complement to the Tb probe (5' ATG ATA TTT TCT TTA 3') labelled at its 3' with BHQ1 (Biosearch Technologies), 10 µl of supernatant from the 95°C incubation, and as a control 0.4 ng/µl genomic DNA sample purified from a volunteer's whole blood. The supernatant from the automated release was analysed by adding 10 µl of the reaction mixture to the final concentration described above.

The PCR program was as follows: Pre-heating +95 °C 1 min; 35 cycles of following +95 °C 30 s, ramping from +95 °C to +84 C 0.2 °C/s, ramping from +84 °C to +56 °C 2.5 °C/s, +56 °C 1 min, +66 °C 1 min and final extension 7 min. After amplification, the reaction was incubated at +30 °C for 5 min, whereafter the time-resolved fluorescence was measured with Victor2 1420 Multilabel Counter (Wallac).

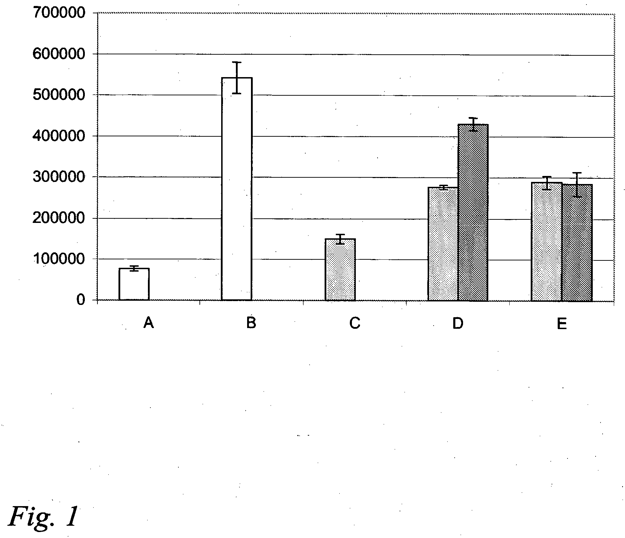

The results of this experiment are shown in Figure 1 by the average and standard deviation of time-resolved Tb fluorescence of each reaction in duplicates. Negative and positive PCR controls are drawn by bars A and B, respectively. When the simplified release (D and E) is compared to elution by manufacturer's recommendation (C), it is clearly seen that the analysis can be performed with blood (black bars) and saliva (grey bars) disks without prewash both at room temperature (D) and at +95 C (E). The result from the supernatants is also comparable to the estimated amount of genomic DNA per blood disk.

### Example 2

### DNA analysis from whole blood and saliva

This example shows how the pre-treatment of blood and saliva samples can be simplified to enable simple automation. The sample is collected onto protein denaturing matrix, and the release of nucleic acids from the matrix, amplification and detection are all performed in a single reaction vial. After release, the amplification and detection reagents are added and the reaction vial is sealed, and after amplification and short analytical incubation, the fluorescence is measured without opening the reaction vial, i.e., without removal of the matrix.

First, whole blood and saliva was collected from two volunteers. The samples were applied onto IsoCode^{®} (Schleicher & Schuell) collection paper and allowed to dry. For analysis, a 1.2-mm blood disk or two 1.2-mm saliva disks were punched and the nucleic acids were released at room temperature for 30 min in 40 µl of distilled water. The release was performed in PCR vials and as a reference in separate micro tubes. After release, the PCR/detection reaction mixture was added into the PCR vial comprising the disk. As reference, the 40-µl supernatant from the separate tubes was analysed.

The closed-tube PCR amplification and detection reaction was performed in a total volume of 50 µl. A 10-µl portion of following PCR/detection reaction mixture was added into the PCR: 5 x DyNAzyme™ buffer (Finnzymes), 1 mM dNTP's, 12.5 mM MgCl2, 1 µM CFTR exon 10 forward primer (5' AAG CAC AGT GGA AGA ATT TC 3'), 0.25 µM CFTR exon 10 reverse primer (5' CTC TTC TAG TTG GCA TGC T 3'), 0.1 U/µl DyNAzyme™ enzyme (Finnzymes), 83 nM analyte specific oligonucleotide probe (5' ACC AAA GAT-GAT ATT TAA A 3') labelled at its 5' end with a stable fluorescent W8184 Eu chelate (Wallac) and 166 nM quencher probe complement to the Eu probe (5' TCA TTG GTG TTT 3') labelled at its 3' with BHQ1 (Biosearch Technologies). As a control, 20 ng/reaction of genomic DNA sample purified from a volunteer's whole blood was analysed.

The PCR program was as follows: Pre-heating 95°C 1 min; 35 cycles of following +95 °C 30 s, +56 °C 1 min, +66 °C 1 min, final extension 7 min and final denaturation 8 min. After amplification, the reaction was incubated at +40 °C for 20 min and at +22 °C for 15 min, whereafter the time-resolved fluorescence was measured with Victor2 1420 Multilabel Counter (Wallac) directly from the unopened vial.

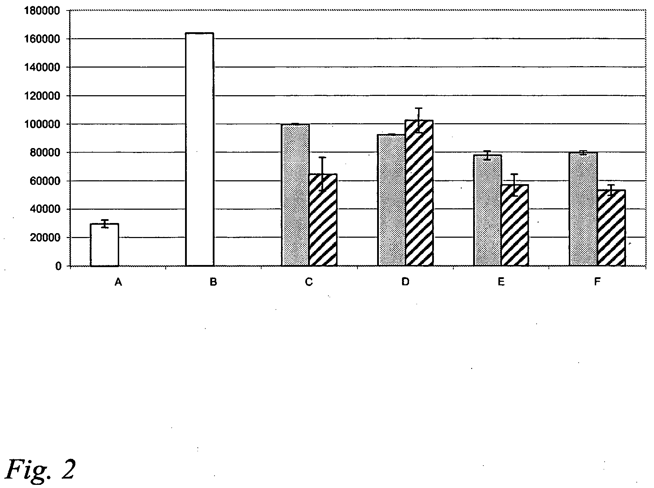

The results of this experiment are shown in Figure 2 by the average and standard deviation of time-resolved Eu fluorescence of each reaction in triplicates. Negative and positive PCR controls are drawn by bars A and B, respectively. C-D represent results from blood and E-F from saliva samples obtained from 2 volunteers. When the integrated release in the reaction vial without removal of matrix at any point (hatched bars) is compared to release in a separate vial (grey bars), it is clearly seen that the whole analysis can be performed with blood disk and saliva disks without removal of disk. The result shows that the release from the matrix can be performed at room temperature in the reaction vial and after amplification the detection can be performed without removal of matrix enabling simple automation of whole analysis.

### Example 3

### Integrated DNA analysis from whole blood and saliva

This example shows how the release of nucleic acids from matrix can be further simplified to enable simple automation. The sample is collected onto protein denaturing matrix, and the release of nucleic acids, amplification and detection are all performed in a single reaction vial even in the presence of amplification and detection reagents. The matrix comprising the sample is added together with the amplification/detection reagents into a reaction vial, the vial is sealed, and after amplification and short analytical incubation, the fluorescence is measured without opening the reaction vial, i.e. without removal of the matrix.

First, whole blood and saliva was collected from two volunteers. The samples were applied onto IsoCodeⓇ (Schleicher & Schuell) collection paper and allowed to dry.

For analysis, a 1.2-mm blood disk or two 1.2-mm saliva disks were punched into PCR vials. The closed-tube PCR amplification and detection reaction was performed in a total volume of 50 µl in following PCR/detection reaction mixture: 1 x HotStarTaq® buffer (Qiagen), 0.2 mM dNTP's, 2.5 mM MgCl2, 0.05 % BSA, 0.2 µM CFTR exon 10 forward primer (5' AAG CAC AGT GGA AGA ATT TC 3'), 0.05 µM CFTR exon 10 reverse primer (5' CTC TTC TAG TTG GCA TGC T 3'), 0.02 U/pl DyNAzyme™ enzyme (Finnzymes), 17 nM analyte specific oligonucleotide probe (5' ACC AAA GAT GAT ATT TAA A 3') labelled at its 5' end with a stable fluorescent W8184 Eu chelate (Wallac) and 33 nM quencher probe complement to the Eu probe (5' TCA TTG GTG TTT 3') labelled at its 3' with BHQ1 (Biosearch Technologies). As a control, 20 ng/reaction of a volunteer's genomic DNA sample on disk was analysed. As a reference, the release of nucleic acids was performed at room temperature for 30 min in 40 µl of distilled water, and analysed in the closed-tube PCR/detection after addition of a 10-µl portion of reaction mixture as 5 x concentrate.

The PCR program was as follows: Pre-heating +95 °C 15 min; 35 cycles of following +95 °C 30 s, +56 °C 1 min, +66 °C 1 min, final extension 7 min and final denaturation 8 min. After amplification, the reaction was incubated at +40 °C for 20 min and at +22 °C for 15 min, whereafter the time-resolved fluorescence was measured with Victor2 1420 Multilabel Counter (Wallac).

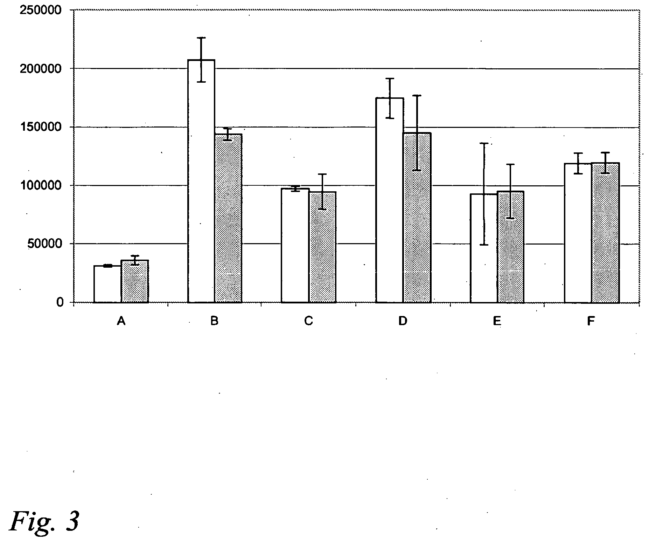

The results of this experiment are shown in Figure 3 by the average and standard deviation of time-resolved Eu fluorescence of each reaction in triplicates. Negative and positive PCR controls are drawn by bars A and B, respectively. C-D represent results from blood and E-F from saliva samples obtained from 2 volunteers. When the release in amplification/detection reagents (grey bars) is compared to release in distilled water (white bars), the result is nearly equal with both methods. The result shows that release from the matrix can be performed even in the amplification/detection mixture, and the whole process can be performed in a single vial in a closed-tube format enabling simple automation of whole analysis.

### Example 4

### Integrated sample pre-treatment and DNA analysis from whole blood

This example shows how the matrix comprising the sample can be used in analysis right after the application of the sample enabling development of amplification/detection devices comprising the matrix in the reaction vial. The sample is applied onto the protein denaturing absorbent matrix in the reaction vial, the amplification/detection reagents are added, the vial is sealed, and after amplification and short analytical incubation, the fluorescence is measured without opening the reaction vial.

The closed-tube PCR amplification and detection reaction was performed in a total volume of 50 µl. First, a 1.2-mm disk from IsoCode® (Schleicher & Schuell) collection paper was punched into PCR vials. Then, a 0.35-µl sample from a volunteer's whole blood was applied into the vial. After various time points, a 50-µl portion of PCR/detection reaction mixture (as follows) was added: 1 x Phusion™ HF buffer (Finnzymes), 0.2 mM dNTP's, 2.5 mM MgCl2, 0.02 % BSA, 0.2 µM CFTR exon 10 forward primer (5' AAG CAC AGT GGA AGA ATT TC 3'), 0.05 µM CFTR exon 10 reverse primer (5' CTC TTC TAG TTG GCA TGC T 3'), 0.02 U/µl DyNAzyme™ enzyme (Finnzymes), 17 nM analyte specific oligonucleotide probe (5' ACC AAA GAT GAT ATT TAA A 3') labelled at its 5' end with a stable fluorescent W8184 Eu chelate (Wallac) and 33 nM quencher probe complement to the Eu probe (5' TCA TTG GTG TTT 3') labelled at its 3' with BHQ1 (Biosearch Technologies). As a control, 20 ng/reaction of a volunteer's genomic DNA sample on disk was analysed.

The PCR program was as follows: Pre-heating +95 C 15 min; 35 cycles of following +95 °C 30 s, +56 °C 1 min, +66 °C 1 min, final extension 7 min and final denaturation 8 min. After amplification, the reaction was incubated at +40 °C for 20 min and at +22 °C for 15 min, whereafter the time-resolved fluorescence was measured with Victor2 1420 Multilabel Counter (Wallac).

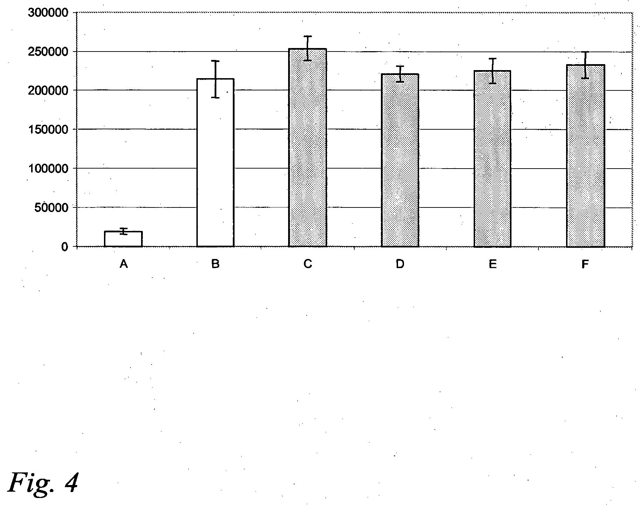

The results of this experiment are shown in Figure 4 by the average and standard deviation of time-resolved Eu fluorescence of each reaction in 4 replicates. Negative and positive PCR controls are drawn by bars A and B, respectively. C-F represent results from blood samples analysed. The signal from the analysis which has been performed right after applying the sample into the amplification/detection vial comprising the matrix (F) is equal to those which have been let dry 1, 2 and 16 h (C-E, respectively). The result shows that the whole process can be performed in a single vial avoiding multiple steps from sample pre-treatment to detection allowing very simple automation.

### Example 5

### Integrated DNA analysis from whole blood using dried reagents

This example shows the extreme simplification of the nucleic acid analysis process from sample pre-treatment to detection. The reagents are dried into the reaction vial, and the sample is either applied onto the protein denaturing absorbent matrix in the vial, or the matrix containing sample is punched into the vial together with water. Then the vial is sealed, and after amplification and short analytical incubation, the fluorescence is measured without opening the vial.

First, a 12.5-µl portion of following amplification/detection reaction mixture was allowed to dry in the reaction vial at room temperature for 2 h: 4 x Phusion™ HF buffer (Finnzymes), 0.8 mM dNTP's, 10 mM MgCl2, 0.08 % BSA, 0.8 µM CFTR exon 10 forward primer (5' AAG CAC AGT GGA AGA ATT TC 3'), 0.2 µM CFTR exon 10 reverse primer (5' CTC TTC TAG TTG GCA TGC T 3'), 0.08 U/µl DyNAzyme™ enzyme (Finnzymes), 66 nM analyte specific oligonucleotide probe (5' ACC AAA GAT GAT ATT TAA A 3') labelled at its 5' end with a stable fluorescent W8184 Eu chelate (Wallac) and 133 nM quencher probe complement to the Eu probe (5' TCA TTG GTG TTT 3') labelled at its 3' with BHQ1 (Biosearch Technologies).

Then, the reaction vial comprising the dried reagents was used in the closed-tube PCR amplification and detection reaction in a total volume of 50 µl. A 1.2-mm blood disk of previously collected volunteer's sample on IsoCode® (Schleicher & Schuell) was punched into the reaction vial with simultaneous dispensing of 50 µl of distilled water. Or, a 1.2-mm disk from IsoCode® (Schleicher & Schuell) collection paper was punched into the vial, and then, a 0.35-µl sample from a volunteer's whole blood was applied into the vial, and 50 µl of distilled water was added. Also, supernatant after release of nucleic acids in 50 µl of distilled water in a separate vial at room temperature for 30 min was used as sample in analysis. As a positive control, 20 ng/reaction of a volunteer's genomic DNA sample on disk was analysed.

The PCR program was as follows: Pre-heating +95 °C 15 min; 35 cycles of following +95 °C 30 s, +56 °C 1 min, +66 C 1 min, final extension 7 min and final denaturation 8 min. After amplification, the reaction was incubated at +40 °C for 20 min and at +22 °C for 15 min, whereafter the time-resolved fluorescence was measured with Victor2 1420 Multilabel Counter (Wallac).

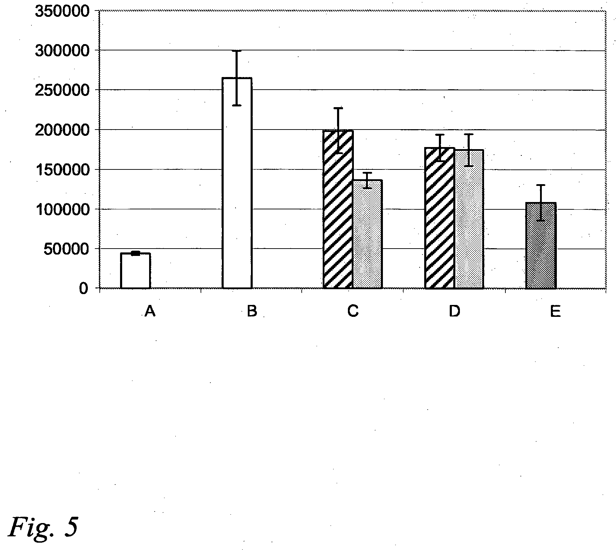

The results of this experiment are shown in Figure 5 by the average and standard deviation of time-resolved Eu fluorescence of each reaction in 4 replicates. Negative and positive PCR controls are drawn by bars A and B, respectively. C-E represent the results from blood samples obtained from 3 volunteers, respectively. When the integrated release in the reaction vial (hatched bars) is compared to the release in a separate vial (grey bars), it is clearly seen that the whole analysis can be performed using dried reagents even without removal of disk. Also, E shows that the matrix can be integrated into the reaction vial comprising the dried reagents, and the sample can be applied right before the analysis. The result shows that the whole process from sample pre-treatment to detection can be performed in a single reaction vial comprising all reagents dried enabling extremely simple automation.

## Claims

1. A method for analyzing a target nucleic acid in a biological sample, comprising the following steps
i) transferring an absorbent matrix to a reaction vial;
ii) providing a biological sample containing said target nucleic acids on, or applying it onto, said absorbent matrix;
iii) providing in said reaction vial a reagent mixture for amplifying said nucleic acids;
iv) providing in said reaction vial a detection reagent mixture;
v) sealing said reaction vial;
vi) performing an amplification reaction; and
vii) detecting the amplified nucleic acids using fluorescence resonance energy transfer, wherein
said biological sample is provided without prior extraction, washing, or elution of the nucleic acids, and
steps vi) and vii) are performed in said vial without removing said matrix from the vial, and wherein the detection step is performed in closed-tubes, without opening the seals.

2. The method according to claim 1, wherein the sample is provided on the absorbent matrix, and at least a portion of said matrix is transferred to the vial together with said sample.

3. The method according to claim 2, wherein the sample is applied onto the matrix immediately prior to transferring said matrix to the reaction vial, without drying and/or storage.

4. The method of claims 1, wherein the matrix is transferred to the reaction vial prior to step ii) and the sample is applied onto said matrix contained in said vial.

5. The method according to claims 1-4, wherein the matrix is an absorbent material that does not irreversibly bind nucleic acids.

6. The method according to claim 5, wherein the matrix comprises a protein binding and/or a protein denaturing agent.

7. The method according to claim 5, wherein the matrix comprises a chaotropic agent.

8. The method according to claims 1-7, wherein at least part of the amplification and detection reagent mixtures are contained in the reaction vial in dried form, said method including an optional further step of adding water prior to step v).

9. The method according to claims 1-8, wherein the amplification is performed by PCR.

10. The method according to claims 1-9, wherein the detection step is performed by hybridization of the amplified sample to a fluorescently labelled probe.

11. A kit for use in a method according to claims 1-10, comprising at least one reaction vial comprising: an absorbent matrix; and a reagent mixture for amplifying said nucleic acids; and a detection reagent mixture for detecting said amplified nucleic acids; wherein at least part of said reagent and detection mixtures are provided in dried form.

12. A kit according to claim 11, wherein all reagents are provided in dried form in said reaction vial.

## Patentansprüche

1. Verfahren zum Analysieren einer Ziel-Nukleinsäure in einer biologischen Probe, folgende Schritte umfassen:
i) Überführen einer Absorptionsmatrix in ein Reaktionsfläschchen;
ii) Bereitstellen einer biologischen Probe, die die Ziel-Nukleinsäuren enthält, an der Absorptionsmatrix oder Aufbringen derselben auf die Absorptionsmatrix;
iii) Bereitstellen eines Reaktionsgemischs in dem Reaktionsfläschchen zum Amplifizieren der Nukleinsäuren;
iv) Bereitstellen eines Nachweisreagensgemischs in dem Reaktionsfläschchen;
v) dichtes Verschließen des Reaktionsfläschchens;
vi) Ausführen einer Amplifikationsreaktion und
vii) Nachweisen der amplifizierten Nukleinsäuren mit Hilfe von Fluoreszenz-Resonanzenergietransfer, wobei
die biologische Probe ohne vorherige Extraktion, Waschung oder Elution der Nukleinsäuren bereitgestellt wird, und
Schritte vi) und vii) in dem Reaktionsfläschchen ausgeführt werden, ohne die Matrix aus dem Fläschchen zu entfernen, und wobei der Nachweisschritt in verschlossenen Röhrchen ohne Öffnen der Abdichtungen ausgeführt wird.

2. Verfahren nach Anspruch 1, wobei die Probe an der Absorptionsmatrix bereitgestellt wird und mindestens ein Teil der Matrix zusammen mit der Probe in das Fläschchen überführt wird.

3. Verfahren nach Anspruch 2, wobei die Probe auf die Matrix aufgebracht wird, unmittelbar bevor die Matrix in das Reaktionsfläschchen überführt wird, ohne Trocknung und/oder Aufbewahrung.

4. Verfahren nach Anspruch 1, wobei die Matrix vor Schritt ii) in das Reaktionsfläschchen überführt wird und die Probe auf die in dem Fläschchen enthaltene Matrix aufgebracht wird.

5. Verfahren nach Anspruch 1 bis 4, wobei die Matrix ein Absorptionsmaterial ist, das Nukleinsäuren nicht irreversibel bindet.

6. Verfahren nach Anspruch 5, wobei die Matrix ein Protein bindendes und/oder ein Protein denaturierendes Mittel umfasst.

7. Verfahren nach Anspruch 5, wobei die Matrix ein chaotropes Mittel umfasst.

8. Verfahren nach Anspruch 1 bis 7, wobei mindestens ein Teil des Amplifikations- und des Nachweisreagensgemischs in dem Reaktionsfläschchen in trockener Form enthalten sind, wobei das Verfahren einen optionalen weiteren Schritt des Zugebens von Wasser vor Schritt v) enthält.

9. Verfahren nach Anspruch 1 bis 8, wobei die Amplifikation durch PCR ausgeführt wird.

10. Verfahren nach Anspruch 1 bis 9, wobei der Nachweisschritt durch Hybridisierung der amplifizierten Probe an eine fluoreszierend markierte Sonde ausgeführt wird.

11. Kit zur Anwendung in einem Verfahren nach Anspruch 1 bis 10, umfassend mindestens ein Reaktionsfläschchen, das umfasst: eine Absorptionsmatrix; und ein Reagensgemisch zum Amplifizieren der Nukleinsäuren; und ein Nachweisreagensgemisch zum Nachweisen der amplifizierten Nukleinsäuren; wobei mindestens ein Teil des Reagens- und des Nachweisgemischs in trockener Form bereitgestellt sind.

12. Kit nach Anspruch 11, wobei alle Reagenzien in trockener Form in dem Reaktionsfläschchen bereitgestellt sind.

## Revendications

1. Procédé pour analyser un acide nucléique cible dans un échantillon biologique, comprenant les étapes suivantes
i) transfert d'une matrice absorbante vers une fiole de réaction ;
ii) fourniture d'un échantillon biologique contenant lesdits acides nucléiques cibles sur, ou application de ce dernier sur, ladite matrice absorbante ;
iii) fourniture dans ladite fiole de réaction d'un mélange de réactifs pour amplifier lesdits acides nucléiques ;
iv) fourniture dans ladite fiole de réaction d'un mélange de réactifs de détection ;
v) cachetage de ladite fiole de réaction ;
vi) exécution d'une réaction d'amplification ; et
vii) détection des acides nucléiques amplifiés en utilisant un transfert d'énergie de fluorescence par résonance, dans lequel
ledit échantillon biologique est fourni sans extraction, lavage, ou élution préalables des acides nucléiques, et
les étapes vi) et vii) sont effectuées dans ladite fiole sans enlever ladite matrice de la fiole, et dans lequel l'étape de détection est effectuée dans des tubes fermés, sans ouvrir les cachets.

2. Procédé selon la revendication 1, dans lequel l'échantillon est fourni sur la matrice absorbante, et au moins une partie de ladite matrice est transférée vers la fiole en même temps que ledit échantillon.

3. Procédé selon la revendication 2, dans lequel l'échantillon est appliqué sur la matrice immédiatement avant de transférer ladite matrice vers la fiole de réaction, sans séchage et/ou stockage.

4. Procédé selon la revendication 1, dans lequel la matrice est transférée vers la fiole de réaction avant l'étape ii) et l'échantillon est appliqué sur ladite matrice contenue dans ladite fiole.

5. Procédé selon les revendications 1 à 4, dans lequel la matrice est une matière absorbante qui ne lie pas de manière irréversible des acides nucléiques.

6. Procédé selon la revendication 5, dans lequel la matrice comprend une liaison protéique et/ou un agent de dénaturation de protéine.

7. Procédé selon la revendication 5, dans lequel la matrice comprend un agent chaotrope.

8. Procédé selon les revendications 1 à 7, dans lequel au moins une partie des mélanges de réactif d'amplification et de détection est contenue dans la fiole de réaction sous forme séchée, ledit procédé incluant une étape supplémentaire facultative d'ajout d'eau avant l'étape v).

9. Procédé selon les revendications 1 à 8, dans lequel l'amplification est effectuée par PCR.

10. Procédé selon les revendications 1 à 9, dans lequel l'étape de détection est effectuée par hybridation de l'échantillon amplifié à une sonde étiquetée de manière fluorescente.

11. Kit à utiliser dans un procédé selon les revendications 1 à 10, comprenant au moins une fiole de réaction comprenant : une matrice absorbante ; et un mélange de réactifs pour amplifier lesdits acides nucléiques ; et un mélange de réactifs de détection pour détecter lesdits acides nucléiques amplifiés ; dans lequel au moins une partie desdits mélanges de réactif et de détection est fournie sous forme séchée.

12. Kit selon la revendication 11, dans lequel tous les réactifs sont fournis sous forme séchée dans ladite fiole de réaction.
